# FASCICULE DE BREVET EUROPEEN

(11) **EP 0 925 777 B1**
(45) Date de publication et mention de la délivrance du brevet: **03.01.2001**
(21) Numéro de dépôt: 98402775.5
(22) Date de dépôt: 09.11.1998
(51) Int. Cl.: A61K 7/48, A61K 7/50

(54) **Composition cosmétique et/ou dermatologique à base d'acide ascorbique sous forme de poudre**
Pulverförmige ascorbinsaüre enthaltende kosmetische und/oder dermatologische zusammensetzung
Cosmetic and/or dermatologic composition containing powdery ascorbic acid

(30) Priorité: 15.12.1997 FR 9715860
(43) Date de publication de la demande: 30.06.1999
(73) Titulaire: L'OREAL, 75008 Paris (FR)
(72) Inventeur: Jacquier, Isabelle, 75012 Paris (FR)
(74) Mandataire: Rasson, Catherine

(56) Documents cités:
- US-A- 4 913 896
- US-A- 5 472 688
- STN, Serveur de Bases de Données, XP002067107 & JP 01 079105 A (SAITO)
- STN, Serveur de Bases de Données, XP002067108 & JP 02 169508 A (KOBAYASHI KOSE CO.)

## Description

L'invention se rapporte à une composition cosmétique et/ou dermatologique se présentant sous forme d'une poudre et comportant de l'acide ascorbique pulvérulent, de l'amidon et au moins un tensioactif anionique. Cette composition est notamment utile pour le nettoyage de la peau aussi bien du corps que du visage, y compris autour des yeux et sur le cuir chevelu.

L'invention se rapporte aussi à une utilisation de cette composition pour le nettoyage et/ou le traitement de la peau, ainsi qu'à un procédé pour le nettoyage et/ou le traitement cosmétique de la peau.

On cherche depuis longtemps à formuler l'acide ascorbique ou vitamine C dans les domaines cosmétique et dermatologique, sous différentes formes galéniques, du fait de ses nombreuses propriétés bénéfiques. En particulier, l'acide ascorbique stimule la synthèse du tissu conjonctif et notamment du collagène, renforce les défenses du tissu cutané contre les agressions extérieures telles que les rayonnements ultraviolets et la pollution, compense la déficience en vitamine E de la peau, dépigmente la peau et possède une fonction anti-radicaux libres.

Malheureusement, en raison de sa structure chimique (d'alpha-cétolactone), l'acide ascorbique est très sensible à certains paramètres de l'environnement comme la lumière, l'oxygène et l'eau. Il s'ensuit donc une dégradation rapide de l'acide ascorbique formulé lorsqu'il est en contact avec un de ces paramètres, ce qui va à l'encontre de l'efficacité recherchée.

Par ailleurs, pour utiliser les effets bénéfiques de l'acide ascorbique dans une composition de nettoyage de la peau, il faut aussi que la composition le contenant ait de bonnes propriétés moussantes sans que cela ne déstabilise l'acide ascorbique.

Pour diminuer et/ou retarder la dégradation de l'acide ascorbique, plusieurs solutions ont déjà été envisagées dans l'état de la technique. L'une des solutions consiste notamment à utiliser l'acide ascorbique sous forme de poudre et à le dissoudre dans de l'eau juste avant l'emploi. Ainsi, le document JP-A-01079105 décrit une poudre à base d'acide ascorbique pour application topique. Toutefois, du fait de l'acidité élevée de la solution obtenue, celle-ci est agressive et risque de provoquer des irritations importantes de la peau. En outre, une telle composition ne présente pas de propriétés moussantes.

Il subsiste donc le besoin d'une composition contenant de l'acide ascorbique, pouvant être stockée plusieurs années avant d'être utilisée, tout en présentant de bonnes propriétés moussantes à l'utilisation.

La demanderesse a maintenant trouvé une composition cosmétique et/ou dermatologique surmontant les inconvénients de l'art antérieur.

La présente invention a pour objet une composition cosmétique et/ou dermatologique se présentant sous forme d'une poudre et comportant de l'acide ascorbique pulvérulent, de l'amidon et au moins un tensioactif anionique.

La composition se présente sous forme d'une poudre, c'est-à-dire sous forme d'un solide finement divisé en particules ayant une dimension moyenne de particules, allant par exemple de 50 à 600 µm et de préférence de 150 à 400 µm.

La poudre obtenue est préparée par simple mélange des constituants pulvérulents ; elle est donc facile à fabriquer. En outre, elle a une excellente solubilité dans l'eau et a de bonnes propriétés moussantes. Par ailleurs, elle est agréable à utiliser sur la peau du fait de sa douceur.

Comme acide ascorbique pulvérulent, on peut utiliser par exemple l'acide ascorbique pulvérulent vendu par HOFFMAN-LA ROCHE. L'acide ascorbique est présent dans la composition de l'invention en une quantité allant par exemple de 0,1 à 20 % et de préférence de 0,5 à 10 % du poids total de la composition.

L'amidon peut être notamment choisi parmi l'amidon de riz, l'amidon de maïs, l'amidon de blé, l'amidon de pomme de terre, l'amidon d'avoine, l'amidon de tapioca et leurs mélanges. Il peut être naturel ou modifié.

Selon un mode préféré de réalisation de l'invention, l'amidon compris dans la composition de l'invention est un amidon modifié, et en particulier de l'amidon réticulé. Cet amidon modifié peut être par exemple choisi parmi les amidons réticulés par un groupe fonctionnel, par exemple les amidons réticulés par l'anhydride octénylsuccinique et plus particulièrement le "Aluminium Starch octenyl succinate", tel que le produit vendu par la société NATIONAL STARCH sous le nom de DRY-FLO, ou l'amidon de maïs réticulé vendu sous le nom RESISTAMYL E2 par la société AMYLUM ; les amidons modifiés au niveau du rapport amylose/amylopectine tels que le produit HYLON VII vendu par la société AMYLUM ; les amidons modifiés tels que l'amidon de maïs faiblement quaternisé vendu sous le nom MYPLUS W7 par la société AMYLUM, l'amidon de pomme de terre vendu sous le nom SUPRAMYL P 60 par la société AMYLUM ou l'amidon de maïs hydroxypropylé vendu sous le nom MERIGEL EF6 par la société AMYLUM.

L'amidon est présent dans la composition selon l'invention dans la composition selon l'invention, dans une quantité qui peut varier dans une large mesure. Ainsi, cette quantité peut aller par exemple de préférence en une quantité allant de 10 à 90 % et plus particulièrement de 30 à 60 % du poids total de la composition.

La nature du tensioactif anionique utilisé dans la composition selon l'invention ne revêt pas, dans le cadre de la présente invention, de caractère véritablement critique.

Ainsi, à titre d'exemple de tensioactifs anioniques utilisables, seuls ou mélanges, dans le cadre de la présente invention, on peut citer notamment les sels (en particulier sels alcalins, notamment de sodium, sels d'ammonium, sels d'amines, sels d'aminoalcools ou sels de magnésium) des composés suivants : alkylsulfates, alkyléthersulfates, alkylamidoéther-sulfates, alkylarylpolyéthersulfates, monoglycérides sulfates, alkylsulfonates, alkyl-phosphates, alkylamidesulfonates, alkylarylsulfonates, alpha-oléfine-sulfonates, paraffine-sulfonates, alkylsulfosuccinates, alkyléthersulfosuccinates, alkylamide-sulfosuccinates, alkylsulfosuccinamates, alkylsulfoacétates, alkyléther-phosphates, acylsarcosinates, acyliséthionates et N-acyltaurates, le radical alkyle ou acyle de ces différents composés comportant de préférence de 12 à 20 atomes de carbone, et le radical aryle désignant de préférence un groupement phényle ou benzyle.

Parmi les tensioactifs anioniques encore utilisables, on peut également citer les sels d'acides gras tels que les sels des acides oléique, ricinoléique, palmitique, stéarique et des acides d'huile de coprah ou d'huile de coprah hydrogénée ; les acyl-lactylates dont le radical acyle comporte 8 à 20 atomes de carbone.

Parmi ces tensioactifs anioniques, on préfère utiliser plus particulièrement les sels d'iséthionate tels que le cocoyl isethionate de sodium ; les sels de glutamate tels que le lauroyl glutamate de sodium et le produit vendu sous le nom "Amisoft HS-11" par la société Ajinomoto (nom CTFA : "sodium hydrogenated tallow glutamate) ; les sels de sarcosinate tels que le mélange sarcosinate de stéaroyle / sarcosinate de myristyle ; les sels d'alkyl sulfate tels que le lauryl sulfate de sodium et ses dérivés ; les sels d'alkyl éther sulfate tels que le laureth sulfate de sodium ; les sels de sulfosuccinate tels que le laureth sulfosuccinate disodique et le MEA sulfosuccinate disodique ; les sels de taurate tels que le methyl cocoyl taurate de sodium ; les sels d'alpha-oléfines sulfonate tels que le C₁₄-C₁₇ alkyl-sec-sulfonate de sodium ; les sels d'alaninate tels que le lauroyl methylamino propionate de sodium ; le lauryl sulfoacétate de sodium, ainsi que leurs mélanges.

Le tensioactif anionique est présent dans la composition selon l'invention dans la composition selon l'invention, dans une quantité qui peut varier dans une large mesure. Ainsi, cette quantité peut aller par exemple de 10 à 80 % et de préférence de 30 à 50 % du poids total de la composition.

Selon un mode particulier de réalisation de l'invention, la composition comprend, en outre au moins un polymère hydrosoluble. Ce polymère hydrosoluble peut être notamment choisi parmi les polysaccharides, les polymères synthétiques, les celluloses et les argiles.

A titre d'exemple de polymères hydrosolubles utilisables dans l'invention, on peut citer les gommes de guar, de xanthane, de carraghénane, de cellulose, de sclerotium, les dérivés de ces gommes, les hydroxyalkylcelluloses, les carboxycelluloses de sodium, les polyacrylamides et les copolymères d'acrylamides, la gélatine, l'agar-agar, les polymères carboxyvinyliques (carbomer), la montmorillonite et le silicate de magnésium et aluminium.

La quantité de polymère dans la composition de l'invention peut aller par exemple de 1 à 5 % et de préférence de 2 à 3 % du poids total de la composition.

La composition de l'invention peut contenir des additifs autres que ceux cités précédemment et généralement utilisés dans les domaines cosmétique et dermatologique, tels que, par exemple, les séquestrants, les parfums, les antioxydants, les actifs hydrosolubles, les conservateurs, les matières colorantes (comme les pigments et les colorants hydrophiles) et les charges minérales et/ou organiques. Ces additifs peuvent être présents dans la composition finale en une quantité de 0 à 50 %, de préférence de 0,5 à 20 % et encore plus particulièrement entre 0,5 et 10 % du poids total de la composition.

Bien entendu, l'homme de l'art veillera à choisir ce ou ces éventuels additifs et/ou leurs quantités de manière telle que les propriétés avantageuses attachées intrinsèquement à la composition conforme à l'invention ne soient pas, ou substantiellement pas, altérées par la ou les adjonctions envisagées.

Comme actifs, on peut citer par exemple les adoucissants tels que l'allantoïne, les hydratants tels que la glycérine et les sucres, les enzymes, les vitamines, les exfoliants tels que les poudres de polyéthylène et la poudre de noyaux d'abricot.

Comme charges, on peut citer par exemple les talcs ou silicates de magnésium hydratés, les micas ou aluminosilicates, les argiles, le kaolin ou silicate d'aluminium hydraté, les nitrures de bore, les polymères et copolymères d'acide acrylique ou méthacrylique tels que les produits vendus sous le nom de "Polytrap" par la société DOW CORNING.

La composition de l'invention est préparée par mélange de tous les constituants dans un homogénéisateur, après éventuellement broyage des poudres ayant une granulométrie trop élevée.

De façon avantageuse, la composition de l'invention comporte un milieu physiologiquement acceptable pour la peau, les muqueuses, les ongles, les fibres kératiniques et/ou les cheveux.

La composition de l'invention donne lieu à la formation de mousse en présence d'eau et présente notamment une bonne efficacité pour le nettoyage de la peau et dans toutes les applications de la vitamine C, notamment pour traiter la peau et en particulier pour la tonifier, la régénérer, pour traiter les rides et/ou les ridules de la peau, pour éclaircir le teint, pour éliminer les taches pigmentaires de la peau, pour lutter contre les méfaits des rayonnements UV et/ou pour renforcer les tissus cutanés contre les agressions de l'environnement.

Aussi, la présente invention a aussi pour objet l'utilisation cosmétique de la composition selon l'invention pour nettoyer la peau, pour traiter la peau et en particulier pour la tonifier, la régénérer, pour traiter les rides et/ou les ridules de la peau, pour éclaircir le teint, pour éliminer les taches pigmentaires de la peau, pour lutter contre les méfaits des rayonnements UV et/ou pour renforcer les tissus cutanés contre les agressions de l'environnement.

La présente invention a aussi pour objet un procédé cosmétique de nettoyage et/ou de traitement de la peau consistant à utiliser la composition selon l'invention.

Selon un mode particulier de réalisation de l'invention, la composition de l'invention est incorporée dans une éponge en présentation unidose. On mouille l'éponge, puis on la passe sur le visage et on rince.

On donne, ci-après, des exemples de compositions conformes à l'invention. Les quantités sont données en pourcentage pondéral.

### Exemple 1 : composition de nettoyage

- Sodium hydrogenated tallow glutamate 11 %
- Cocoyl iséthionate de sodium 28 %
- Aluminium starch octenyl succinate 52,3 %
- Allantoïne 0,2 %
- Acrylates copolymer (POLYTRAP) 2 %
- Parfum 1,5 %
- Acide ascorbique 5 %

La composition obtenue se présente sous forme de poudre ayant une dimension moyenne de particules d'environ 250 µm. On mouille la poudre avant de l'appliquer sur la peau, puis on rince cette dernière.

### Exemple 2 : composition de nettoyage

- Acide ascorbique 5 %
- Gomme de xanthane 3 %
- Sodium hydrogenated tallow glutamate 11 %
- Cocoyl iséthionate de sodium 28 %
- Aluminium starch octenyl succinate 49,3 %
- Allantoïne 0,2 %
- Acrylates copolymer (POLYTRAP) 2 %
- Parfum 1,5 %

La composition obtenue se présente sous forme de poudre ayant une dimension moyenne de particules d'environ 250 µm. On mouille la poudre avant de l'appliquer sur la peau, puis on rince cette dernière.

## Revendications

1. Composition cosmétique et/ou dermatologique se présentant sous forme d'une poudre obtenue par simple mélange des constituants pulvérulents et comportant de l'acide ascorbique pulvérulent, de 30 à 90 % en poids d'amidon par rapport au poids total de la composition, et au moins un tensioactif anionique.

2. Composition selon la revendication 1, caractérisée en ce que l'acide ascorbique est présent en une quantité allant de 0,1 % à 20 % et de préférence de 0,5 % à 10 % du poids total de la composition.

3. Composition selon la revendication 1 ou 2, caractérisée en ce que l'amidon est un amidon modifié.

4. Composition selon la revendication précédente, caractérisée en ce que l'amidon modifié est réticulé.

5. Composition selon la revendication 3 ou 4, caractérisée en ce que l'amidon modifié est réticulé par l'anhydride octénylsuccinique.

6. Composition selon l'une quelconque des revendications précédentes, caractérisée en ce que l'amidon est présent en une quantité allant de 30 à 60 % du poids total de la composition.

7. Composition selon l'une quelconque des revendications précédentes, caractérisée en ce que le tensioactif anionique est choisi parmi les sels des alkylsulfates, alkyléthersulfates, alkylamidoéther-sulfates, alkylarylpolyéthersulfates, monoglycérides sulfates, alkylsulfonates, alkylphosphates, alkylamidesulfonates, alkylarylsulfonates, alpha-oléfine-sulfonates, paraffine-sulfonates, alkylsulfosuccinates, alkyléthersulfosuccinates, alkylamidesulfosuccinates, alkylsulfosuccinamates, alkylsulfoacétates, alkylétherphosphates, acylsarcosinates, acyliséthionates et N-acyltaurates, les sels d'acides gras, et leurs mélanges.

8. Composition selon l'une quelconque des revendications précédentes, caractérisée en ce que le tensioactif anionique est choisi parmi le cocoyl isethionate de sodium, le lauroyl glutamate de sodium, le "sodium hydrogenated tallow glutamate, le mélange sarcosinate de stéaroyle / sarcosinate de myristyle, le lauryl sulfate de sodium et ses dérivés, le laureth sulfate de sodium, le laureth sulfosuccinate disodique, le MEA sulfosuccinate disodique, le methyl cocoyl taurate de sodium, le C₁₄-C₁₇ alkyl-sec-sulfonate de sodium, le lauroyl methylamino propionate de sodium, le lauryl sulfoacétate de sodium, et leurs mélanges.

9. Composition selon l'une quelconque des revendications précédentes, caractérisée en ce qu'elle comprend en outre au moins un polymère hydrosoluble.

10. Composition selon la revendication précédente, caractérisée en ce que le polymère hydrosoluble est choisi parmi les polysaccharides, les polymères synthétiques, les celluloses, les argiles et leurs mélanges.

11. Composition selon la revendication 9 ou 10, caractérisée en ce que le polymère hydrosoluble est choisi parmi les gommes de guar, de xanthane, de carraghénane, de cellulose, de sclerotium, les dérivés de ces gommes, les hydroxyalkylcelluloses, les carboxycelluloses de sodium, les polyacrylamides et les copolymères d'acrylamides, la gélatine, l'agar-agar, les polymères carboxyvinyliques, la montmorillonite, le silicate de magnésium et aluminium, et leurs mélanges.

12. Composition selon l'une quelconque des revendications 9 à 11, caractérisée en ce que le polymère est présent en une quantité allant de 1 à 5 % et de préférence de 2 à 3 % du poids total de la composition.

13. Composition selon l'une quelconque des revendications précédentes, caractérisée en ce qu'elle contient en outre au moins un additif choisi parmi les séquestrants, les parfums, les antioxydants, les actifs hydrosolubles, les conservateurs, les matières colorantes et les charges.

14. Composition selon l'une quelconque des revendications précédentes, caractérisée en ce qu'elle est contenue dans une éponge.

15. Utilisation cosmétique de la composition selon l'une quelconque des revendications 1 à 14, pour nettoyer la peau, pour traiter la peau et en particulier pour la tonifier, la régénérer, pour traiter les rides et/ou les ridules de la peau, pour éclaircir le teint, pour éliminer les taches pigmentaires de la peau, pour lutter contre les méfaits des rayonnements UV et/ou pour renforcer les tissus cutanés contre les agressions de l'environnement.

16. Procédé cosmétique de nettoyage et/ou de traitement de la peau, caractérisé en ce qu'il consiste à appliquer sur la peau, y compris autour des yeux, une composition selon l'une quelconque des revendications 1 à 13.

17. Procédé selon la revendication 16, caractérisé en ce que la composition est contenue dans une éponge.

## Claims

1. Cosmetic and/or dermatological composition in the form of a powder obtained by simply mixing the pulverent constituents and containing pulverulent ascorbic acid, from 30 to 90% by weight of starch relative to the total weight of the composition and at least one anionic surfactant.

2. Composition according to Claim 1, characterized in that the ascorbic acid is present in an amount ranging from 0.1% to 20%, and preferably from 0.5% to 10%, of the total weight of the composition.

3. Composition according to Claim 1 or 2, characterized in that the starch is a modified starch.

4. Composition according to the preceding claim, characterized in that the modified starch is crosslinked.

5. Composition according to Claim 3 or 4, characterized in that the modified starch is crosslinked with octenylsuccinic anhydride.

6. Composition according to any one of the preceding claims, characterized in that the starch is present in an amount ranging from 30 to 60% of the total weight of the composition.

7. Composition according to any one of the preceding claims, characterized in that the anionic surfactant is chosen from salts of alkyl sulphates, alkyl ether sulphates, alkylamido ether sulphates, alkylarylpolyether sulphates, monoglyceride sulphates, alkyl sulphonates, alkyl phosphates, alkylamide sulphonates, alkylaryl sulphonates, a-olefin sulphonates, paraffin sulphonates, alkyl sulphosuccinates, alkyl ether sulphosuccinates, alkylamide sulphosuccinates, alkyl sulphosuccinamates, alkyl sulphoacetates, alkyl ether phosphates, acyl sarcosinates, acyl isethionates and N-acyl taurates, fatty acid salts, and mixtures thereof.

8. Composition according to any one of the preceding claims, characterized in that the anionic surfactant is chosen from sodium cocoyl isethionate, sodium lauroyl glutamate, sodium hydrogenated tallow glutamate, the stearoyl sarcosinate/myristyl sarcosinate mixture; sodium lauryl sulphate and its derivatives, sodium laureth sulphate, disodium laureth sulphosuccinate, disodium MEA sulphosuccinate, sodium methyl cocoyl taurate, sodium C₁₄-C₁₇ sec-alkyl sulphonate, sodium lauroyl methylaminopropionate, sodium lauryl sulphoacetate, and mixtures thereof.

9. Composition according to any one of the preceding claims, characterized in that it also comprises at least one water-soluble polymer.

10. Composition according to the preceding claim, characterized in that the water-soluble polymer is chosen from polysaccharides, synthetic polymers, celluloses and clays, and mixtures thereof.

11. Composition according to Claim 9 or 10, characterized in that the water-soluble polymer is chosen from guar gum, xanthan gum, carrageenan gum, cellulose gum, sclerotium gum, derivatives of these gums, hydroxyalkylcelluloses, sodium carboxycelluloses, polyacrylamides and acrylamide copolymers, gelatin, agar-agar, carboxyvinyl polymers, montmorillonite and magnesium aluminium silicate, and mixtures thereof.

12. Composition according to any one of Claims 9 to 11, characterized in that the polymer is present in an amount ranging from 1 to 5%, and preferably from 2 to 3%, of the total weight of the composition.

13. Composition according to any one of the preceding claims, characterized in that it also contains at least one additive chosen from sequestering agents, fragrances, antioxidants, water-soluble active agents, preserving agents, dyestuffs and fillers.

14. Composition according to any one of the preceding claims, characterized in that it is contained in a sponge.

15. Cosmetic use of the composition according to any one of Claims 1 to 14 for cleansing the skin, for treating the skin and in particular for toning and regenerating it, for treating wrinkles and/or fine lines on the skin, for lightening the complexion, for removing skin pigmentation marks, for combating the harmful effects of UV radiation and/or for reinforcing skin tissues against environmental attacking factors.

16. Cosmetic process for cleansing and/or treating the skin, characterized in that it consists in applying a composition according to any one of Claims 1 to 13 to the skin, including the area around the eyes.

17. Process according to Claim 16, characterized in that the composition is contained in a sponge.

## Patentansprüche

1. Kosmetische und/oder dermatologische Zusammensetzung, die in Form eines Pulvers vorliegt, durch einfaches Mischen der pulverförmigen Bestandteile hergestellt wird und pulverförmige Ascorbinsäure, 30 bis 90 Gew.-% Stärke, bezogen auf das Gesamtgewicht der Zusammensetzung, und mindestens einen anionischen grenzflächenaktiven Stoff enthält.

2. Zusammensetzung nach Anspruch 1, dadurch gekennzeichnet, daß die Ascorbinsäure in einem Mengenanteil von 0,1 bis 20 % und vorzugsweise von 0,5 bis 10 % des Gesamtgewichts der Zusammensetzung vorliegt.

3. Zusammensetzung nach Anspruch 1 oder 2, dadurch gekennzeichnet, daß die Stärke eine modifizierte Stärke ist.

4. Zusammensetzung nach dem vorhergehenden Anspruch, dadurch gekennzeichnet, daß die modifizierte Stärke vernetzt ist.

5. Zusammensetzung nach Anspruch 3 oder 4, dadurch gekennzeichnet, daß die modifizierte Stärke mit Octenylbernsteinsäureanhydrid vernetzt ist.

6. Zusammensetzung nach einem der vorhergehenden Ansprüche, dadurch gekennzeichnet, daß die Stärke in einem Mengenanteil von 30 bis 60 % des Gesamtgewichts der Zusammensetzung vorliegt.

7. Zusammensetzung nach einem der vorhergehenden Ansprüche, dadurch gekennzeichnet, daß der anionische grenzflächenaktive Stoff unter den Salzen von Alkylsulfaten, Alkylethersulfaten, Alkylamidoethersulfaten, Alkylarylpolyethersulfaten, Monoglyceridsulfaten, Alkylsulfonaten, Alkylphosphaten, Alkylamidsulfonaten, Alkylarylsulfonaten, α-Olefinsulfonaten, Paraffinsulfonaten, Alkylsulfosuccinaten, Alkylethersulfosuccinaten, Alkylamidsulfosuccinaten, Alkylsulfosuccinamaten, Alkylsulfoacetaten, Alkyletherphosphaten, Acylsarcosinaten, Acylisethionaten, N-Acyltauraten und Fettsäuren sowie deren Gemischen ausgewählt ist.

8. Zusammensetzung nach einem der vorhergehenden Ansprüche, dadurch gekennzeichnet, daß der anionische grenzflächenaktive Stoff unter Natriumcocoylisethionat, Natriumlauroylglutamat, Sodium hydrogenated tallow glutamate, dem Gemisch von Stearoylsarcosinat/Myristylsarcosinat, Natriumlaurylsufat und seinen Derivaten, Natriumlaurethsulfat, Dinatriumlaurethsulfosuccinat, Dinatrium-MEA-sulfosuccinat, Natriummethylcocoyltaurat, Natrium-C₁₄₋₁₇-sek-alkyl-sulfonat, Natriumlauroylmethylaminopropionat, Natriumlaurylsulfoacetat und deren Gemischen ausgewählt ist.

9. Zusammensetzung nach einem der vorhergehenden Ansprüche, dadurch gekennzeichnet, daß sie ferner mindestens ein wasserlösliches Polymer enthält.

10. Zusammensetzung nach dem vorhergehenden Anspruch, dadurch gekennzeichnet, daß das wasserlösliche Polymer unter den Polysacchariden, synthetischen Polymeren, Celluloseverbindungen, Tonen und deren Gemischen ausgewählt ist.

11. Zusammensetzung nach Anspruch 9 oder 10, dadurch gekennzeichnet, daß das wasserlösliche Polymer unter Guargummi, Xanthangummi, Carrageenangummi, Cellulosegummi, Sclerotiumgummi, Derivaten dieser Gummen, Hydroxyalkylcellulosen, Natriumcarboxycellulosen, Polyacrylamiden und Acrylamid-Copolymeren, Gelatine, Agar-Agar, Carboxyvinylpolymeren, Montmorillonit, Magnesiumaluminiumsilicat und deren Gemischen ausgewählt ist.

12. Zusammensetzung nach einem der Ansprüche 9 bis 11, dadurch gekennzeichnet, daß das Polymer in einem Mengenanteil von 1 bis 5 % und vorzugsweise 2 bis 3 % des Gesamtgewichts der Zusammensetzung vorliegt.

13. Zusammensetzung nach dem vorhergehenden Anspruch, dadurch gekennzeichnet, daß sie ferner mindestens einen Zusatzstoff enthält, der unter den Maskierungsmitteln, Parfums, Antioxidantien, wasserlöslichen Wirkstoffen, Konservierungsmitteln, Färbemitteln und Füllstoffen ausgewählt ist.

14. Zusammensetzung nach dem vorhergehenden Anspruch, dadurch gekennzeichnet, daß sie in einem Schwamm enthalten ist.

15. Kosmetische Verwendung der Zusammensetzung nach einem der Ansprüche 1 bis 14 zur Reinigung und Behandlung der Haut, insbesondere um die Haut zu beleben und zu regenerieren, die Falten und/oder Fältchen der Haut zu behandeln, den Teint strahlender erscheinen zu lassen, die Pigmentflecken der Haut zu entfernen, die schädlichen Wirkungen der UV-Strahlung zu bekämpfen und/oder das Hautgewebe gegen Umwelteinflüsse zu schützen.

16. Kosmetisches Verfahren zur Reinigung und/oder Behandlung der Haut, dadurch gekennzeichnet, daß es darin besteht, eine Zusammensetzung nach einem der Ansprüche 1 bis 13 auf die Haut, einschließlich der Hautpartien um die Augen, aufzubringen.

17. Verfahren nach Anspruch 16, dadurch gekennzeichnet, daß die Zusammensetzung in einem Schwamm enthalten ist.
